# FASCICULE DE BREVET EUROPEEN

(11) **EP 1 898 847 B1**
(45) Date de publication et mention de la délivrance du brevet: **03.07.2013**
(21) Numéro de dépôt: 06764627.3
(22) Date de dépôt: 15.05.2006
(51) Int. Cl.: A61F 2/95

(54) **NECESSAIRE DE LARGAGE D'UN ORGANE DE TRAITEMENT D'UNE CAVITE**
SATZ ZUM EINFÜHREN EINES HOHLRAUMBEHANDLUNGSELEMENTS
KIT FOR INSERTING A CAVITY-TREATMENT ELEMENT

(30) Priorité: 19.05.2005 FR 0505044
(43) Date de publication de la demande: 19.03.2008
(73) Titulaire: Laboratoires Perouse, 60173 Ivry Le Temple (FR)
(72) Inventeur: STYRC, Mikolaj Witold, L-8190 Kopstal (LU)
(74) Mandataire: Domenego, Bertrand
(86) Numéro de dépôt international: PCT/FR2006/001088
(87) Numéro de publication internationale: WO 2006/123046

(56) Documents cités:
- EP-A- 0 472 731
- WO-A-00/27309
- WO-A-02/49538
- WO-A-96/32078

## Description

La présente invention concerne un nécessaire de traitement d'un réseau de circulation du sang, du type comprenant :
- au moins un organe de traitement implantable déformable radialement entre un état rétracté et un état déployé ;
- un fourreau de maintien de l'organe de traitement dans son état rétracté ;
- des moyens d'introduction de l'organe de traitement dans le fourreau par traction.

Ce nécessaire est utilisé notamment pour le largage d'endoprothèses dans un vaisseau sanguin.

On connaît de EP 0 472 731 un nécessaire du type précité, dans lequel une endoprothèse dans un état déployé est introduite dans un fourreau de maintien de cette endoprothèse dans un état rétracté.

Les moyens d'introduction de l'endoprothèse dans le fourreau comprennent un fil de traction d'une extrémité de l'endoprothèse et un entonnoir monté à l'extrémité du fourreau qui permet de contacter l'endoprothèse lorsque celle-ci est tirée par le fil à l'intérieur du fourreau.

Un tel dispositif ne donne pas entière satisfaction. En effet, la force de traction à appliquer sur le fil pour introduire l'endoprothèse dans son fourreau est très élevée. Cette introduction doit donc être réalisée avant le conditionnement du nécessaire, et non lors d'une opération chirurgicale, juste avant l'implantation de l'endoprothèse.

Un but de l'invention est donc de proposer un nécessaire de traitement d'un réseau de circulation du sang dans lequel l'introduction d'un organe de traitement dans un fourreau de maintien de cet organe dans une position rétractée est réalisable facilement, notamment lors d'une opération chirurgicale. Par ailleurs, le nécessaire doit permettre une extraction facile de l'organe de traitement lors de son implantation, en limitant le risque de blessure du patient.

A cet effet, l'invention a pour objet un nécessaire de traitement du type précité, caractérisé en ce que les moyens d'introduction comprennent au moins une chemise de réception de l'organe de traitement déplaçable à coulissement par rapport au fourreau entre une position dilatée de réception de l'organe de traitement hors du fourreau et une position comprimée d'interposition entre l'organe de traitement et le fourreau, l'organe de traitement étant sensiblement fixe axialement par rapport à la chemise de réception lors du déplacement de la chemise de réception entre ses positions de réception et d'interposition et en ce que la chemise comprend au moins un brin de retour retroussé à l'extérieur du fourreau, les moyens d'introduction comprenant un organe de traction du ou de chaque brin retroussé vers une extrémité proximale du fourreau, l'organe de traction étant déplaçable à coulissement le long du fourreau lors du déplacement de la chemise de sa position de réception à sa position d'interposition.

Le nécessaire selon l'invention peut comporter une ou plusieurs des caractéristiques suivantes, prises isolément ou selon toutes les combinaisons techniquement possibles :
- la chemise de réception comprend une région de retenue d'une extrémité de l'organe de traitement et une région de guidage de l'organe de traitement lorsque la région de retenue est disposée dans le fourreau et la région de guidage fait saillie hors du fourreau ;
- l'organe de traitement est fixé de manière libérable aux moyens d'introduction ;
- les moyens d'introduction forment des moyens d'extraction de l'organe de traitement hors du fourreau ;
- la chemise de réception comprend au moins deux lames flexibles liées ensemble par une base commune et présentant des extrémités libres disjointes ;
- les moyens d'introduction comprennent un organe de commande du déplacement de la chemise de réception par rapport au fourreau, l'organe de commande et la chemise de réception étant venus de matière ;
- le coefficient de frottement entre la chemise de réception et le fourreau est inférieur au coefficient de frottement entre l'organe de traitement et le fourreau ;
- l'organe de traitement est une endoprothèse comprenant une armature déployable ; et
- l'organe de traitement est une valve déployable ;
- la chemise comprend, dans sa position de réception, une extrémité de retenue de l'endoprothèse, l'organe de traction étant solidaire d'une extrémité libre de la chemise ;
- l'organe de traction comprend un collier monté à coulissement à l'extérieur du fourreau ;
- l'organe de traction est lié en translation à l'extrémité de retenue ;
- l'organe de traction est disposé dans le fourreau, le fourreau comprenant au moins un passage latéral dans lequel l'extrémité libre de la chemise est engagée ; et
- chaque lame flexible est retroussée autour d'une région distale du fourreau délimitée par un passage latéral et une extrémité distale du fourreau.

L'invention sera mieux comprise à la lecture de la description qui va suivre, donnée uniquement à titre d'exemple et faite en se référant aux dessins annexés sur lesquels :
- la Figure 1 est une vue en élévation d'un premier nécessaire conditionné dans un emballage pour lequel une protection n'est pas demandée ;
- la Figure 2 est une vue en élévation du nécessaire dans une première position de la chemise de réception ;
- la Figure 3 est une vue en coupe suivant un plan médian du nécessaire dans une deuxième position de la chemise de réception ;
- la Figure 4 est une vue analogue à la Figure 2 pour un deuxième nécessaire de traitement pour lequel une protection n'est pas demandée ;
- la Figure 5 est une vue en élévation d'un troisième nécessaire de traitement pour lequel une protection n'est pas demandée ;
- la Figure 6 est une vue analogue à la Figure 2 du troisième nécessaire de traitement selon l'invention ;
- la Figure 7 est une vue analogue à la Figure 3 d'un premier nécessaire de traitement selon l'invention ;
- la Figure 8 est une vue analogue à la Figure 3 d'un deuxième nécessaire de traitement selon l'invention ; et
- la Figure 9 est une vue partielle en perspective du fourreau du deuxième nécessaire selon l'invention représenté sur la Figure 8.

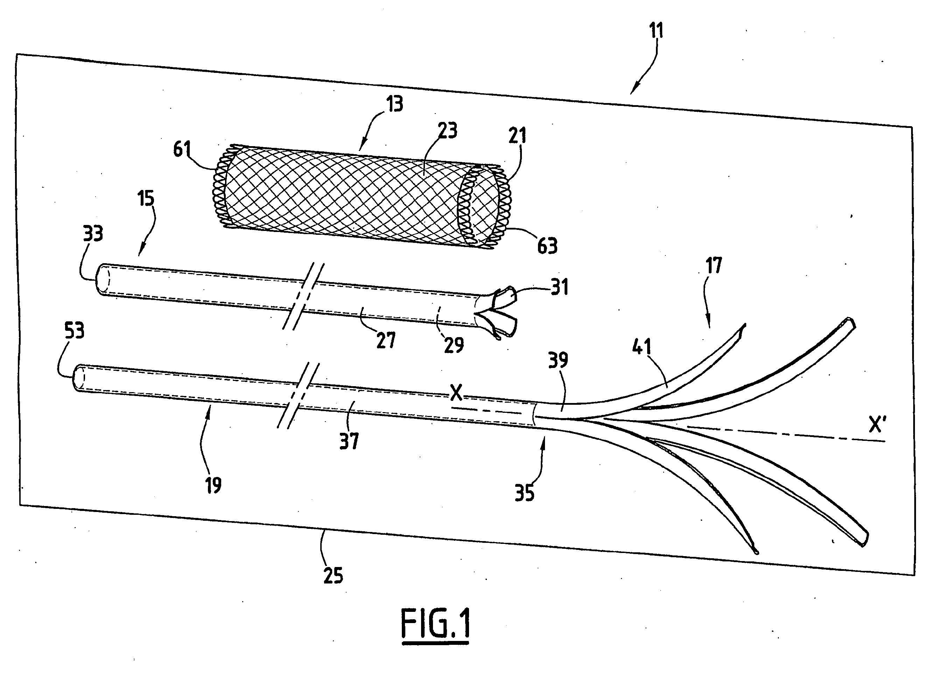

Le nécessaire de traitement 11 représenté sur la Figure 1, qui n'est pas couvert par les revendications de cette demande, comporte une endoprothèse tubulaire 13 déployable entre un état rétracté et un état déployé, un fourreau 15 de maintien de l'endoprothèse 13 dans son état rétracté, et des moyens 19 d'introduction de l'endoprothèse 13 dans le fourreau 15 comportant une chemise 17 de réception de l'endoprothèse 13.

L'endoprothèse 13 comprend un treillis tubulaire 21 en métal implantable tel que de l'acier inoxydable qui possède des propriétés élastiques. Ainsi, dans l'exemple représenté, l'endoprothèse 13 est autoexpansible.

Comme connu en soi, l'endoprothèse 13 est susceptible de se déformer spontanément de son état rétracté, dans lequel elle présente un petit diamètre à son état déployé dans lequel elle présente un diamètre supérieur, cet état déployé constituant son état de repos.

Dans l'exemple illustré, le treillis métallique 21 de l'endoprothèse est noyé dans un film 23 extensible et étanche aux liquides tel qu'un élastomère. Cet élastomère peut être constitué par exemple par une silicone.

Une telle endoprothèse est couramment désignée par le terme anglais « stent ».

Les éléments du nécessaire 11 de traitement sont conditionnés dans un même emballage 25 constitué, par exemple, par une poche hermétique. Dans cet emballage 25, l'endoprothèse 13 est conservée dans son état déployé.

Le fourreau 15 est constitué par un tube 27 en matière plastique, par exemple en polytetrafluoroéthylène (PTFE), délimitant un conduit intérieur 29 de diamètre sensiblement égal au diamètre d'une section transversale de l'endoprothèse 13 dans sa position rétractée.

Le fourreau 15 s'étend longitudinalement entre une extrémité distale 31 destinée à être introduite dans un vaisseau sanguin et une extrémité proximale 33 destinée à être accessible pour un chirurgien. L'extrémité 31 présente une forme conique divergente en bout. Toutefois, en variante l'extrémité 31 est droite.

Dans l'exemple illustré par la Figure 1, les moyens 19 d'introduction de l'endoprothèse 13 dans le fourreau 15 sont formés à partir d'un manchon 35 flexible en polytetrafluoroéthylène (PTFE) dans lequel une pluralité de fentes longitudinales ont été ménagées depuis l'extrémité distale de ce manchon 35.

Le manchon 35 délimite, dans sa partie fendue, la chemise de réception 17 et, dans sa partie pleine, un tube 37 de commande du déplacement de l'endoprothèse.

La chemise de réception 17 de l'endoprothèse 13 comprend une bague 39 de retenue d'une extrémité de l'endoprothèse 13 et une pluralité de lames 41 flexibles de guidage.

La bague de retenue 39 est constituée par l'extrémité de la partie pleine du manchon 35. Elle présente un diamètre extérieur inférieur ou sensiblement égal au diamètre intérieur du fourreau 15.

Les lames flexibles 41 s'étendent à partir de la bague de retenue 39 jusqu'à des extrémités libres disjointes.

Les lames 41 sont délimitées latéralement par les fentes ménagées dans le manchon 39.

Les lames flexibles 41 sont déplaçables entre une position à l'écart d'un axe central X-X' défini par la bague 39 et une position au voisinage de cet axe central X-X'.

La longueur des lames flexibles 41 est au moins sensiblement égale à la longueur de l'endoprothèse 13, de sorte que lorsqu'une extrémité de l'endoprothèse 13 est insérée dans la bague 39, les lames 41, dans leur position au voisinage de l'axe X-X', s'étendent suivant sensiblement toute la longueur de l'endoprothèse 13.

La chemise de réception 17 est déplaçable à coulissement par rapport au fourreau 15 entre une position dilatée de réception de l'endoprothèse 13, hors du fourreau 15, dans laquelle les lames 41 sont disposées à l'écart de l'axe central X-X', une position intermédiaire d'introduction de la bague 39 dans le fourreau 15 et une position comprimée d'interposition dans le fourreau 15 entre l'endoprothèse 13 et le fourreau 15.

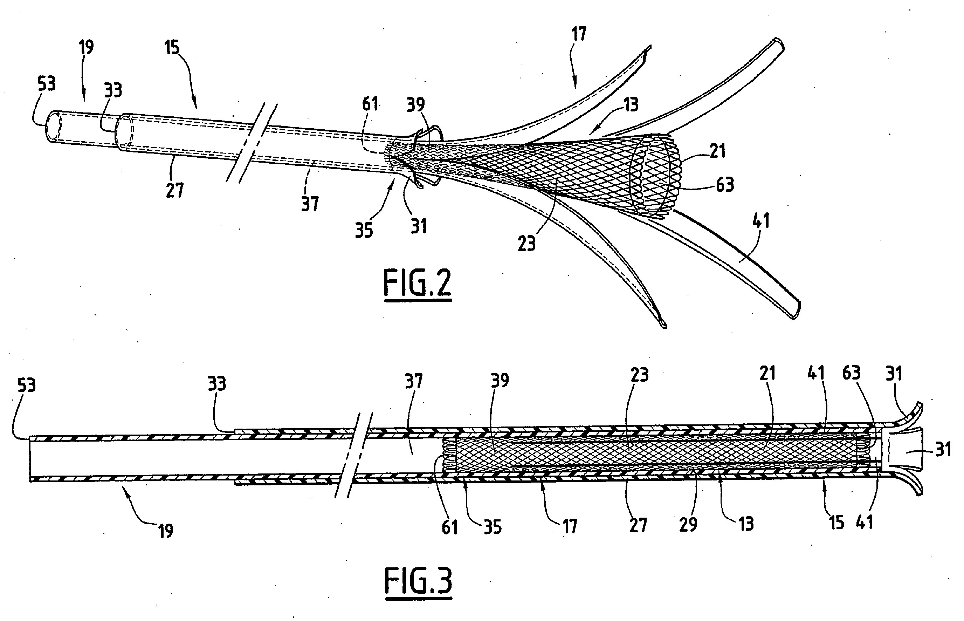

Dans la position intermédiaire d'introduction, illustrée par la Figure 2, la bague de retenue 39 est insérée dans le fourreau 15. Les lames 41 font saillie au moins partiellement à l'extérieur du fourreau 15 et présentent une forme sensiblement divergente vers l'extérieur du fourreau 15. Les lames 41 sont progressivement comprimées vers l'axe X-X' par appui contre la région périphérique à l'extrémité distale 31 du fourreau 15.

Dans la position d'interposition, illustrée par la Figure 3, la chemise 17 contenant l'endoprothèse 13 est sensiblement totalement introduite dans le fourreau 15. L'endoprothèse 13 est maintenue dans son état rétracté et les lames 41 sont plaquées contre le treillis métallique 21 de l'endoprothèse 13 par la paroi interne du fourreau 15.

Le tube de commande 37 présente une longueur supérieure à la longueur du fourreau 15 de sorte que son extrémité proximale 53 fait saillie au-delà de l'extrémité proximale 33 du fourreau 15 lorsque le tube de commande 37 est disposé dans le fourreau 15.

Le tube de commande 37 est déplaçable à coulissement dans le fourreau 15 par traction ou poussée sur son extrémité proximale 53. Ainsi, le tube de commande 37 forme également un moyen d'extraction de l'endoprothèse 13 hors du fourreau 15 lors de son implantation dans un vaisseau sanguin, comme on va le décrire plus bas.

Dans l'exemple illustré, la bague de retenue 39, les lames flexibles 41, et le tube de commande 37 sont venus de matière. En variante, ils sont constitués par des pièces distinctes.

On décrira maintenant comme exemple un procédé de préparation de l'endoprothèse 13 en vue de son implantation dans un vaisseau sanguin, à partir du nécessaire 11 selon l'invention.

Ce procédé comprend une phase de conservation et une phase de préparation.

Dans la phase de conservation, l'endoprothèse 13 est maintenue dans l'emballage 25 dans son état déployé, ce qui permet de préserver les propriétés mécaniques ou morphologiques de l'endoprothèse 13, particulièrement lorsque son treillis tubulaire 21 est noyé dans un film extensible et étanche 23.

Dans la phase de préparation proprement dite, qui est réalisée juste avant l'implantation de l'endoprothèse 13 au cours d'une opération chirurgicale, le chirurgien déchire l'emballage 25 et découpe l'endoprothèse 13 pour adapter sa longueur à la morphologie du vaisseau sanguin dans lequel elle doit être implantée.

Initialement, il monte le tube de commande 37 à coulissement dans le fourreau 15. La chemise 17 est disposée dans sa position de réception de l'endoprothèse 13 dans laquelle la bague 39 et les lames 41 de la chemise de réception 17 font saillie hors du fourreau 15.

Le chirurgien insère alors une extrémité 61 de l'endoprothèse 13 dans la bague 39 de la chemise 17, par exemple en pinçant cette extrémité 61.

L'endoprothèse 13 est alors retenue par serrage dans la bague 39 à l'extrémité du tube de commande 37. Les lames 41 sont disposées en regard de l'armature 21.

En référence à la Figure 2, le chirurgien tire alors sur l'extrémité proximale 53 du tube de commande 37 et déplace cette extrémité 53 à l'écart du fourreau 15. La bague 39, les lames 41 et l'endoprothèse 13 pénètrent alors par coulissement dans le fourreau 15.

Lors de ce déplacement, les lames 41 glissent contre la paroi interne du fourreau 15 et empêchent sensiblement tout contact entre cette paroi interne et l'armature 21 de l'endoprothèse 13. En raison de la forme divergente des lames 41 vers l'extérieur du fourreau 15, les lames 41 sont progressivement comprimées vers l'axe X-X' par appui contre la région périphérique à l'extrémité distale 31 du fourreau 15. Ceci permet la compression progressive de l'endoprothèse 13 depuis son état déployé vers son état rétracté, comme illustré par la Figure 3.

La présence de la chemise de réception 17 lors de l'introduction de l'endoprothèse 13 dans le fourreau 15 diminue sensiblement la force de frottement entre l'endoprothèse 13 et la paroi intérieure du fourreau 15. La force de traction minimale à appliquer sur le tube de commande 37 pour l'introduction de l'endoprothèse 13 dans le fourreau 15 est donc sensiblement égale à la force de frottement entre la chemise de réception 17 et la paroi intérieure du fourreau 15.

Le rapport entre le coefficient de frottement entre l'endoprothèse 13 et la paroi intérieure du fourreau 15 et le coefficient de frottement entre la chemise de réception 17 et la paroi intérieure du fourreau 15 est supérieur à 10 et notamment compris entre 10 et 100.

Ainsi, la force de traction à appliquer sur les moyens d'introduction 19 est considérablement réduite et l'introduction de l'endoprothèse 13 dans le fourreau 15 est donc réalisable très simplement.

Pour déployer l'endoprothèse 13 dans le vaisseau sanguin, le chirurgien implante un guide chirurgical (non représenté) circulant dans le vaisseau sanguin ou la veine depuis le point d'introduction extérieur jusqu'à la zone de la veine ou de l'artère dans laquelle doit être implantée l'endoprothèse 13.

L'endoprothèse 13 est maintenue, dans sa position rétractée par le fourreau 15 est alors introduite jusqu'à son lieu d'implantation par déplacement le long du guide chirurgical.

Une fois l'endoprothèse 13 introduite, le chirurgien déploie l'extrémité distale 63 de l'endoprothèse 13 dans le vaisseau par poussée sur le tube de commande 37 pour déplacer son extrémité proximale 53 vers le fourreau 15.

L'extrémité distale 63 de l'endoprothèse 13 se déploie hors du fourreau en écartant les lames flexibles 41 de sorte que le treillis 21 se rapproche des parois du vaisseau à traiter pour venir en appui sur ces parois.

Lors de l'extraction de l'endoprothèse 13 hors du fourreau 15, la présence de la chemise 17 de réception supprime également sensiblement le frottement entre l'endoprothèse 13 et le fourreau 15, ce qui facilite le déploiement de l'endoprothèse 13 dans le vaisseau.

Une fois l'extrémité distale 63 de l'endoprothèse 13 fixée contre les parois du vaisseau à traiter, le chirurgien procède au retrait de la chemise 17 et du fourreau 15 hors du vaisseau sanguin.

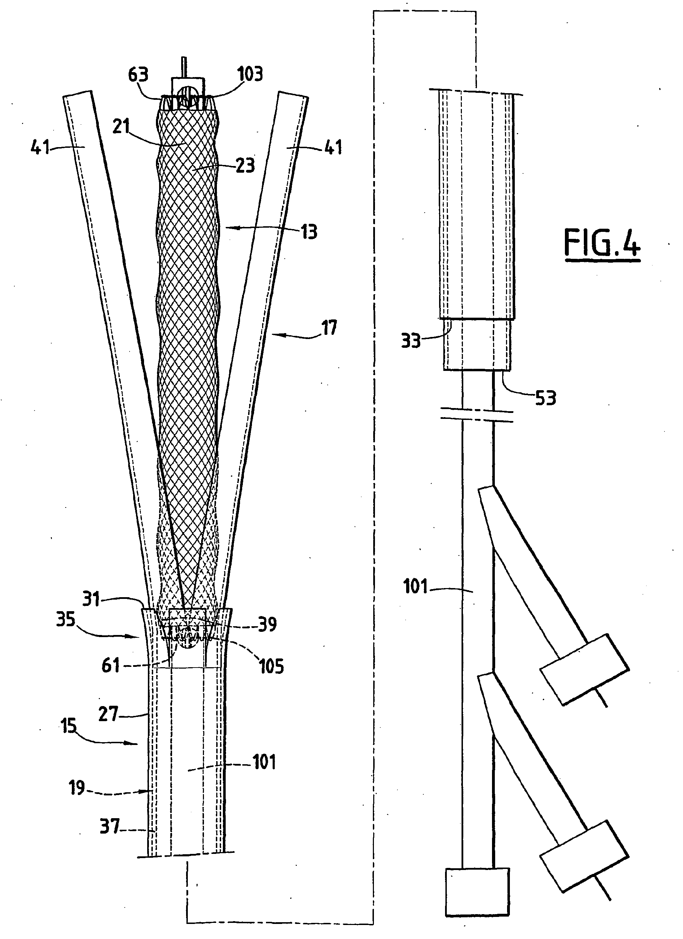

Dans la variante représentée sur la Figure 4, l'endoprothèse 13 est montée de manière coaxiale sur un tuteur 101 unique et est fixée sur ce tuteur 101 par des fils de retenue 103, 105 libérables du type décrit dans la demande française n° 03 14424.

Les fils 103, 105 sont engagés dans l'armature 21 respectivement aux extrémités distale 63 et proximale 61 de l'endoprothèse 13, et forment à ces extrémités 61, 63 des boucles de serrage dont la longueur peut être commandée par des prolongements des fils 103, 105 jusqu'à l'extrémité proximale du tuteur 101.

Le diamètre du tuteur 101 est sensiblement inférieur au diamètre interieur du fourreau 15 et de la bague 39.

Ainsi, lors de la phase de conservation, la chemise de réception 17, le tube de commande 37 engagé dans le fourreau 15, l'endoprothèse 13 dans son état déployé sont disposés coaxialement sur le tuteur 101.

Comme décrit dans la demande française précitée, lors de l'opération chirugicale, le chirurgien serre dans une premier temps les boucles de serrage aux extrémités du treillis 121 par traction sur les prolongements des fils 103, 105, afin d'amener l'endoprothèse 13 dans un état rétracté intermédiaire.

Puis, pour introduire l'endoprothèse 13 dans le fourreau 15, le chirurgien introduit l'extrémité proximale 61 de l'endoprothèse dans la bague 39 puis déplace simultanément les extrémités proximales du tuteur 101 et du tube de commande 37 à l'écart de l'extrémité proximale 33 du fourreau 15.

L'endoprothèse 13 reçue dans la chemise 17 pénètre alors dans le fourreau 15, comme décrit précédemment.

Une fois dans le fourreau 15, l'endoprothèse 13 présente une section transversale de diamètre minimal sur toute sa longueur.

Son déploiement dans le vaisseau sanguin s'effectue comme dans la demande française précitée.

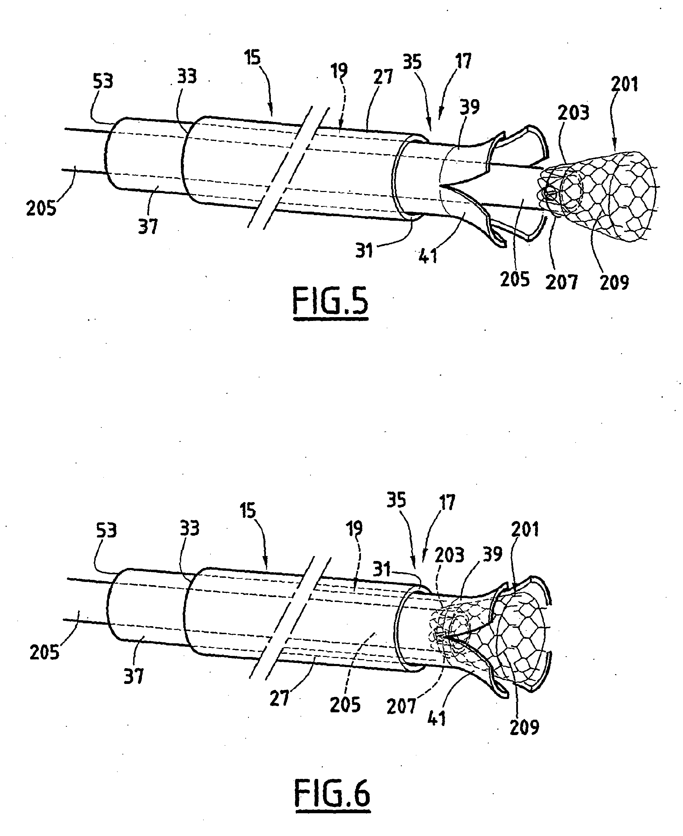

Dans une troisième variante représentée en regard des Figures 5 et 6, le nécessaire comprend une valve cardiaque 201 déployable entre un état rétracté et un état déployé.

Lors de la phase de conservation, la valve 201 dans son état déployé est fixée par son extrémité 203 convergente à une tige d'introduction 205. Cette fixation est réalisée par exemple à l'aide d'un fil de retenue 207 libérable à partir du point d'introduction extérieur dans le réseau sanguin à traiter.

A la différence du premier nécessaire, la chemise de réception 17, puis le fourreau 15 sont montés coaxialement sur la tige d'introduction 205, de sorte que l'extrémité proximale de cette tige 205 fait saillie au-delà du fourreau 15.

Pour préparer la valve 201 en vue de son implantation, le chirurgien déplace l'extrémité proximale de la tige 205 à l'écart de l'extrémité proximale 33 du fourreau 115 par coulissement dans le fourreau 15, en maintenant la chemise de réception 17 sensiblement fixe par rapport au fourreau 15 à l'aide du tube de commande 37.

Le déplacement de la tige 205 permet l'introduction de l'extrémité convergente 203 de la valve 201 dans la bague 39. Dans cette position intermédiaire, illustrée par la Figure 6, les lames flexibles 41 sont disposées en regard du treillis 209 de la valve 201 et présentent alors une forme sensiblement divergente à l'écart du fourreau.

Puis le chirurgien déplace l'extrémité proximale 53 du tube de commande 37 à l'écart du fourreau 15. La valve 201 étant fixée dans la bague 39, le déplacement de l'extrémité proximale de la tige 205 à l'écart du fourreau 15 se poursuit. L'ensemble formé par la valve 201 et sa chemise de réception 17 est alors inséré dans le fourreau 15.

Le déploiement de la valve 201 s'effectue comme décrit précédemment pour l'endoprothèse 13, la dernière étape consistant à libérer le fil de retenue 207 de la valve 201, puis à retirer la tige 205 du réseau de circulation du sang.

Il est donc possible de disposer d'un nécessaire de traitement d'un réseau de circulation du sang, qui permet l'introduction simple et par une force de traction de faible intensité, d'un organe de traitement 13, 201 dans un fourreau de maintien 15 de cet organe 13, 201 dans une position rétractée. Cette introduction est réalisable facilement lors d'une opération chirurgicale.

Par ailleurs, ce nécessaire facilite l'extraction de l'organe de traitement 13, 201 hors du fourreau 15 lors de son implantation dans un réseau de circulation du sang.

Ce nécessaire permet la conservation de l'organe de traitement 13, 201 dans son état déployé, et sa découpe éventuelle juste avant son implantation, afin d'adapter sa longueur à la morphologie du réseau sanguin dans lequel il doit être implanté.

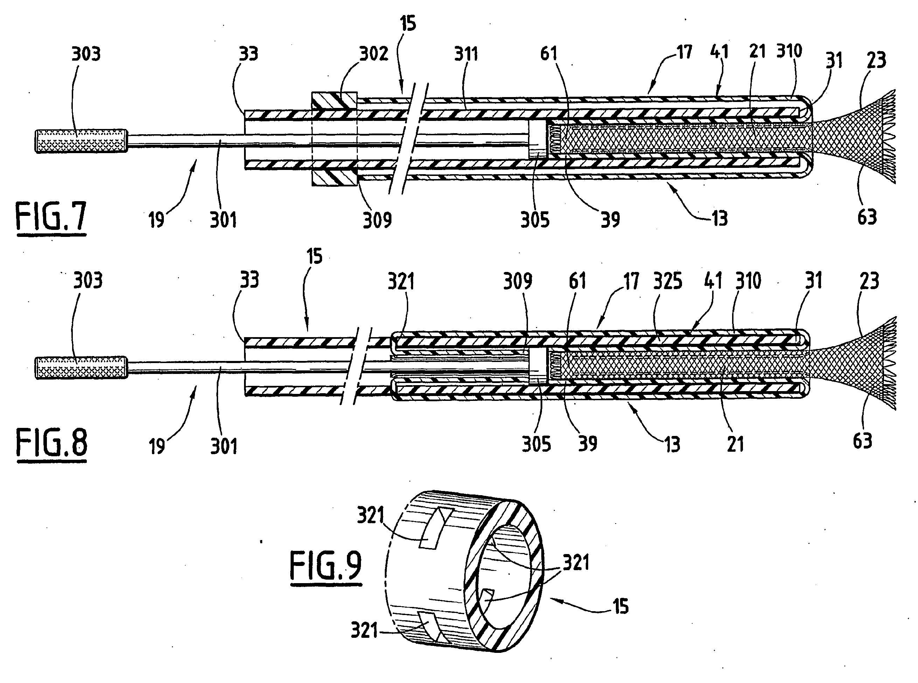

Le premier nécessaire selon l'invention, représenté sur la Figure 7, diffère du nécessaire représenté sur la Figure 3 par les caractéristiques suivantes.

Les moyens d'introduction 19 de l'endoprothèse 13 dans le fourreau 15 comprennent une tige 301 de commande du déplacement de la chemise de réception 17, engagée dans le fourreau 15, et un collier de traction 302 de la chemise 17 monté sur le fourreau 15.

La tige 301 est munie, à son extrémité proximale, d'une poignée 303, et à son extrémité distale, d'un piston 305 de poussée de l'endoprothèse 13.

Le piston 305 est monté à coulissement dans le fourreau 15.

La bague de retenue 39 de l'endoprothèse 13 formée dans la che- . mise de réception 17 est fixée sur une surface distale du piston 305, à l'opposé de la tige 301.

Chaque lame 41 de la chemise 17 comprend un brin de retour 310 en saillie hors du fourreau 15. Les brins de retour 310 sont retroussés contre la surface extérieure notée 311 du fourreau.

Les extrémités libres 309 des lames 41, situées à l'opposé de la bague de retenue 39, sont fixées le long d'une surface périphérique distale du collier 302 de traction.

Le collier 302 est monté à coulissement sur une surface extérieure 311 du fourreau 15. Il est mobile le long du fourreau 15 entre une position proximale de mise en place de l'endoprothèse 13 dans le fourreau 15 dans laquelle le collier est à l'écart de l'extrémité de libération de l'endoprothèse, et une position distale qu'il occupe lorsque l'endoprothèse 13 est totalement introduite dans le fourreau 15 et dans laquelle le collier est plus proche de l'extrémité de libération.

Le collier 302 comprend par ailleurs des moyens de guidage (non représentés) qui empêchent sa rotation autour de l'axe longitudinal du fourreau 15.

La longueur des lames 41 est au moins supérieure à trois fois la longueur de l'endoprothèse 13 de sorte que le collier 302 dans ses positions proximale et distale est disposé à l'extérieur du corps du patient. Pour le reste, les caractéristiques du premier nécessaire selon l'invention sont analogues aux caractéristiques du premier nécessaire décrit sur les Figures 1 à 3.

Initialement, pour introduire l'endoprothèse 21 dans le fourreau 15, le piston 305 est disposé au voisinage de l'extrémité distale 31 du fourreau. La bague de retenue 39 est alors située dans le fourreau 15, au voisinage de l'extrémité distale 31. Le collier 302 est maintenu dans sa position proximale. La position du collier 302 est ajustée manuellement par traction vers l'extrémité proximale 33 du fourreau 15 pour que les brins de retour 310 des lames 41 soient plaqués sur la surface extérieure 311 du fourreau. La dimension transversale maximale du nécessaire est ainsi réduite.

Puis, l'extrémité proximale 61 de l'endoprothèse 13 est introduite dans la bague de retenue 39. Le piston 305 est alors déplacé vers l'extrémité proximale 33 du fourreau 15 par actionnement de la poignée 303.

Lors de ce déplacement, les lames 41 et l'endoprothèse 13 pénètrent dans le fourreau 15. Les lames 41 s'interposent entre l'endoprothèse 13 et le fourreau 15. Par ailleurs, le collier 302 est tiré vers sa position distale par les lames 41 de guidage.

Pour larguer l'endoprothèse 13 dans le patient, le chirurgien déplace simultanément la poignée 303 vers l'extrémité distale 31 du fourreau 15 et le collier 302 vers l'extrémité proximale 33 de ce fourreau 15 pour tirer les brins de retour 310 des lames 41 vers l'extrémité 33.

Ainsi, les lames 41 sont plaquées contre la surface extérieure 311 du fourreau 15, ce qui limite le risque de blessure du patient lors du déploiement de l'endoprothèse, notamment par déchirure de la paroi du vaisseau sanguin.

En variante, le premier nécessaire selon l'invention peut comprendre les caractéristiques du nécessaire de la Figure 4 ou du nécessaire des Figures 5 et 6 à l'exception des lames flexibles. 41 qui sont retroussées à l'extérieur du fourreau.

Le deuxième nécessaire selon l'invention représenté sur la Figure 8 diffère du nécessaire représenté sur la Figure 7 par les caractéristiques suivantes.

Comme illustré par la Figure 9, le fourreau 15 comprend une pluralité de passages transversaux 321 ménagés dans la paroi latérale du fourreau. Ces passages 321 s'étendent sensiblement le long de la périphérie d'une section transversale du fourreau 15. La distance entre l'extrémité distale 31 et les passages 321 est supérieure à la longueur de l'endoprothèse 13.

Le nombre de passages 321 est égal au nombre de lames 41. Par ailleurs, la largeur des passages 321, prise le long de la périphérie est sensiblement égale à la largeur des lames 41.

Les extrémités libres 309 des lames 41 sont engagées à travers les passages 321 et sont fixées sur une surface proximale du piston 305.

Le piston forme ainsi un organe de traction des brins de retour 310.

La longueur des lames 41 est choisie sensiblement égale à deux fois la longueur qui sépare l'extrémité proximale 31 des passages 321. Ainsi, les brins de retour 310 des lames 41 situés hors du fourreau 15 sont plaqués sur la surface extérieure 311 du fourreau 15.

Chaque lame 41 est retroussée autour d'une région distale 325 du fourreau 15 délimitée par un passage 321 et l'extrémité distale 31. Chaque lame 41 forme, autour de cette région, une bande convoyeuse d'introduction de l'endoprothèse 13 dans le fourreau 15.

Ainsi, pour l'introduction de l'endoprothèse 13 dans le fourreau 15, la bague 39 est placée à l'extrémité distale 31 du fourreau 15 par déplacement du piston 305. Puis, l'extrémité proximale 61 de l'endoprothèse 13 est insérée dans la bague 39, et le piston 305 est déplacé vers l'extrémité distale 33 du fourreau 15.

Le déploiement de l'endoprothèse 13 est réalisé par déplacement de ce piston 305 vers l'extrémité proximale 31 du fourreau 15.

## Revendications

1. Nécessaire (11) de traitement d'un réseau de circulation du sang, du type comprenant :
- au moins un organe de traitement (13 ; 201) implantable déformable radialement entre un état rétracté et un état déployé ;
- un fourreau (15) de maintien de l'organe de traitement dans son état rétracté ;
- des moyens (19) d'introduction de l'organe de traitement (13 ; 201) dans le fourreau (15) par traction ;
**caractérisé en ce que** les moyens (19) d'introduction comprennent au moins une chemise (17) de réception de l'organe de traitement (13 ; 201) déplaçable à coulissement par rapport au fourreau (15) entre une position dilatée de réception de l'organe de traitement (13 ; 201) hors du fourreau et une position comprimée d'interposition entre l'organe de traitement (13; 201) et le fourreau (15), l'organe de traitement (13 ; 201) étant sensiblement fixe axialement par rapport à la chemise de réception (17) lors du déplacement de la chemise de réception (17) entre ses positions de réception et d'interposition
et **en ce que** la chemise (17) comprend au moins un brin de retour (310) retroussé à l'extérieur du fourreau (15), les moyens d'introduction (19) comprenant un organe de traction (302 ; 305) du ou de chaque brin retroussé (310) vers une extrémité proximale (33) du fourreau (15), l'organe de traction (302 ; 305) étant déplaçable à coulissement le long du fourreau (15) lors du déplacement de la chemise (17) de sa position de réception à sa position d'interposition.

2. Nécessaire (11) selon la revendication 1, **caractérisé en ce que** la chemise de réception (17) comprend une région de retenue (39) d'une extrémité (61) de l'organe de traitement (13 ; 201) et une région de guidage (41) de l'organe de traitement (13 ; 201) lorsque la région de retenue (39) est disposée dans le fourreau (15) et la région de guidage (41) fait saillie hors du fourreau (15).

3. Nécessaire (11) selon l'une des revendications 1 ou 2, **caractérisé en ce que** l'organe de traitement (13 ; 201) est fixé de manière libérable aux moyens d'introduction (19).

4. Nécessaire (11) selon l'une quelconque des revendications précédentes, **caractérisé en ce que** les moyens d'introduction (19) forment des moyens d'extraction de l'organe de traitement (13 ; 201) hors du fourreau (15).

5. Nécessaire (11) selon l'une quelconque des revendications précédentes, **caractérisé en ce que** la chemise de réception (17) comprend au moins deux lames flexibles (41) liées ensemble par une base commune (39) et présentant des extrémités libres disjointes.

6. Nécessaire (11) selon l'une quelconque des revendications précédentes, **caractérisé en ce que** les moyens d'introduction (19) comprennent un organe (37) de commande du déplacement de la chemise de réception (17) par rapport au fourreau (15), l'organe de commande (37) et la chemise de réception (17) étant venus de matière.

7. Nécessaire (11) selon l'une quelconque des revendications précédentes, **caractérisé en ce que** le coefficient de frottement entre la chemise de réception (17) et le fourreau (15) est inférieur au coefficient de frottement entre l'organe de traitement (13 ; 201) et le fourreau (15).

8. Nécessaire (11) selon l'une quelconque des revendications précédentes, **caractérisé en ce que** l'organe de traitement (13 ; 201) est une endoprothèse (13) comprenant une armature (21) déployable.

9. Nécessaire (11) selon l'une quelconque des revendications précédentes, **caractérisé en ce que** l'organe de traitement (13 ; 201) est une valve déployable (201).

10. Nécessaire (11) selon l'une quelconque des revendications précédentes, **caractérisée en ce que** la chemise (17) comprend, dans sa position de réception, une extrémité (39) de retenue de l'endoprothèse (13), l'organe de traction (302 ; 305) étant solidaire d'une extrémité libre (309) de la chemise (17).

11. Nécessaire (11) selon la revendication 10, **caractérisé en ce que** l'organe de traction comprend un collier (302) monté à coulissement à l'extérieur du fourreau (15).

12. Nécessaire (11) selon la revendication 10, **caractérisé en ce que** l'organe de traction (305) est lié en translation à l'extrémité (39) de retenue.

13. Nécessaire (11) selon la revendication 12, **caractérisé en ce que** l'organe de traction (305) est disposé dans le fourreau (15), le fourreau (15) comprenant au moins un passage latéral (321) dans lequel l'extrémité libre (309) de la chemise (17) est engagée.

14. Nécessaire (11) selon la revendication 13, prise en combinaison avec la revendication 5, **caractérisé en ce que** chaque lame flexible (41) est retroussée autour d'une région distale du fourreau (15) délimitée par un passage latéral (321) et une extrémité distale (31) du fourreau (15).

## Patentansprüche

1. Satz (11) zum Behandeln eines Blutkreislaufnetzes, von einer Art, welche aufweist:
mindestens eine implantierbare Behandlungsvorrichtung (13; 201), welche zwischen einem eingefahrenen und einem entfalteten Zustand radial verformbar ist;
eine Ummantelung (15) zur Beibehaltung der Behandlungsvorrichtung in ihrem eingefahrenen Zustand;
Mittel (19) zum Einführen der Behandlungsvorrichtung (13; 201) durch Zug in die Ummantelung (15);
**dadurch gekennzeichnet, dass** die Einführungsmittel (19) mindestens einen Mantel (17) zur Aufnahme der Behandlungsvorrichtung (13; 201) aufweisen, welcher in Bezug auf die Ummantelung (15) zwischen einer aufgeweiteten Position der Aufnahme der Behandlungsvorrichtung (13; 201) außerhalb der Ummantelung (15) und einer komprimierten Position eines Einfügens zwischen der Behandlungsvorrichtung (13; 201) und der Ummantelung gleitend verlagerbar ist, wobei die Behandlungsvorrichtung axial im Wesentlichen fest ist in Bezug auf den Aufnahmemantel (17) beim Verlagern des Aufnahmemantels (17) zwischen seiner Aufnahme- und seiner Einfügeposition
und dadurch, dass der Mantel (17) mindestens ein Rücklaufteil (310) aufweist, welches außerhalb der Ummantelung (15) ist, wobei die Einführungsmittel (19) eine Zugvorrichtung (302; 305) eines jeden Teils (310) nahe einem proximalen Ende (33) der Ummantelung (15) aufweisen, wobei die Zugvorrichtung (302; 305) längs der Ummantelung (15) gleitend verlagerbar ist während der Verlagerung des Mantels (17) von seiner Aufnahme- zu seiner Einfügeposition.

2. Satz (11) gemäß Anspruch 1, **dadurch gekennzeichnet, dass** der Aufnahmemantel (17) einen Haltebereich (39) eines Endes (61) der Behandlungsvorrichtung (13; 201) und einen Führungsbereich (41) der Behandlungsvorrichtung (13; 201) aufweist, wenn der Haltebereich (39) in der Ummantelung (15) angeordnet ist und der Führungsbereich (41) aus der Ummantelung (15) herausragt.

3. Satz (11) gemäß einem der Ansprüche 1 oder 2, **dadurch gekennzeichnet, dass** die Behandlungsvorrichtung (13; 201) auf lösbare Art an den Einführungsmitteln (19) befestigt ist.

4. Satz (11) gemäß einem der vorhergehenden Ansprüche, **dadurch gekennzeichnet, dass** die Einführungsmittel (19) Extraktionsmittel der Behandlungsvorrichtung (13; 201) aus der Ummantelung (15) bilden.

5. Satz (11) gemäß einem der vorhergehenden Ansprüche, **dadurch gekennzeichnet, dass** der Aufnahmemantel (17) mindestens zwei flexible Teile (41) aufweist, welche miteinander mittels einer gemeinsamen Basis (39) verbunden sind und freie, unverbundene Enden aufweisen.

6. Satz (11) gemäß einem der vorhergehenden Ansprüche, **dadurch gekennzeichnet, dass** die Einführungsmittel (19) eine Steuervorrichtung (37) zum Verlagern des Aufnahmemantels (17) in Bezug auf die Ummantelung (15) aufweisen, wobei die Steuervorrichtung (37) und der Aufnahmemantel (17) einstückig ausgebildet sind.

7. Satz (11) gemäß einem der vorhergehenden Ansprüche, **dadurch gekennzeichnet, dass** der Reibungskoeffizient zwischen dem Aufnahmemantel (17) und der Ummantelung (15) kleiner ist als der Reibungskoeffizient zwischen der Behandlungsvorrichtung (13; 201) und der Ummantelung (15).

8. Satz (11) gemäß einem der vorhergehenden Ansprüche, **dadurch gekennzeichnet, dass** die Behandlungsvorrichtung (13; 201) eine Endoprothese (13) ist, welche ein entfaltbares Teil (21) aufweist.

9. Satz (11) gemäß einem der vorhergehenden Ansprüche, **dadurch gekennzeichnet, dass** die Behandlungsvorrichtung (13; 201) ein Ventil (201) ist.

10. Satz (11) gemäß einem der vorhergehenden Ansprüche, **dadurch gekennzeichnet, dass** der Mantel (17) in seiner Aufnahmeposition ein Halteende (39) der Endoprothese (13) aufweist, wobei die Zugvorrichtung (302; 305) mit dem einen freien Ende (309) des Mantels (17) verbunden ist.

11. Satz (11) gemäß Anspruch 10, **dadurch gekennzeichnet, dass** die Zugvorrichtung eine Schelle (302) aufweist, welche außerhalb der Ummantelung (15) gleitend montiert ist.

12. Satz (11) gemäß Anspruch 10, **dadurch gekennzeichnet, dass** die Zugvorrichtung (305) bei der Translation mit dem Halteende (39) verbunden ist.

13. Satz (11) gemäß Anspruch 12, **dadurch gekennzeichnet, dass** die Zugvorrichtung (305) in der Ummantelung (15) angeordnet ist, wobei die Ummantelung (15) mindestens eine seitliche Öffnung (321) aufweist, in welche das freie Ende (309) des Mantels (17) eingreift.

14. Satz (11) gemäß Anspruch 13 in Verbindung mit Anspruch 5, **dadurch gekennzeichnet, dass** jedes flexible Teil (41) um einen distalen Bereich der Ummantelung (15) herum umgestülpt ist, welcher durch eine seitliche Öffnung (321) und ein distales Ende (31) der Ummantelung (15) begrenzt ist.

## Claims

1. Kit (11) for treating a blood circulation system, comprising:
- at least one implantable treatment element (13; 201) which can be radially deformed between a retracted state and a deployed state;
- a sheath (15) for maintaining the treating element in its retracted state;
- means (19) for pulling the treatment element (13; 201) into the sheath (15);
**characterised in that** the introduction means (19) comprise at least one sleeve (17) for receiving the treatment element (13; 201), which can slide in relation to the sheath (15) between an expanded position for receiving the treatment element (13; 201) outside the sheath and a compressed interposed position between the treatment element (13; 201) and the sheath (15), the treatment element (13; 201) being substantially axially fixed in relation to the receiving sleeve (17) when the receiving sleeve (17) moves between its receiving and interposed positions,
and **in that** the sleeve (17) comprises at least one return strand (310) curved back to the outside of the sheath (15), the introduction means (19) comprising an element (302; 305) for pulling the or each curved strand (310) towards a proximal end (33) of the sheath (15), the pulling element (302; 305) being able to slide along the sheath (15) when the sleeve (17) is displaced between its receiving position and its interposed position.

2. Kit (11) according to claim 1, **characterised in that** the receiving sleeve (17) comprises a region (39) for retaining an end (61) of the treatment element (13; 201) and a region (41) for guiding the treatment element (13; 201) when the retaining region (39) is inside the sheath (15) and the guiding region (41) projects outside the sheath (15).

3. Kit (11) according to either claim 1 or claim 2, **characterised in that** the treatment element (13; 201) is detachably fixed to the introduction means (19).

4. Kit (11) according to any one of the previous claims, **characterised in that** the introduction means (19) form the means for extracting the treatment element (13; 201) from the sheath (15).

5. Kit (11) according to any one of the preceding claims, **characterised in that** the receiving sleeve (17) comprises at least two flexible prongs (41) joined together by a common base (39) and having free ends which are not connected.

6. Kit (11) according to any one of the preceding claims, **characterised in that** the introduction means (19) comprise an element (37) for controlling displacement of the receiving sleeve (17) in relation to the sheath (15), the controlling element (37) and the receiving sleeve (17) being integral.

7. Kit (11) according to any one of the preceding claims, **characterised in that** the coefficient of friction between the receiving sleeve (17) and the sheath (15) is less than the coefficient of friction between the treatment element (13; 201) and the sheath (15).

8. Kit (11) according to any one of the preceding claims, **characterised in that** the treatment element (13; 201) is an endoprosthesis (13) comprising a deployable frame (21).

9. Kit (11) according to any one of the preceding claims, **characterised in that** the treatment element (13; 201) is a deployable valve (201).

10. Kit (11) according to any one of the preceding claims, **characterised in that** the sleeve (17) comprises, in its receiving position, an end (39) for retaining the endoprosthesis (13), the pulling element (302; 305) being integral with a free end (309) of the sleeve (17).

11. Kit (11) according to claim 10, **characterised in that** the pulling element comprises a collar (302) slidingly mounted to the outside of the sheath (15).

12. Kit (11) according to claim 10, **characterised in that** the pulling element (305) is connected in translation to the retaining end (39).

13. Kit (11) according to claim 12, **characterised in that** the pulling element (305) is disposed in the sheath (15), the sheath (15) comprising at least one lateral passage (321) in which the free end (309) of the sleeve (17) is engaged.

14. Kit (11) according to claim 13, taken in combination with claim 5, **characterised in that** each flexible prong (41) is curved around a distal region of the sheath (15) delimited by a lateral passage (321) and a distal end (31) of the sheath (15).
